# EUROPÄISCHE PATENTSCHRIFT

(11) **EP 4 458 343 B1**
(45) Veröffentlichungstag und Bekanntmachung des Hinweises auf die Patenterteilung: **09.04.2025**
(21) Anmeldenummer: 23171450.2
(22) Anmeldetag: 04.05.2023
(51) Int. Cl.: A61J 1/20, A61J 1/14, A61M 5/162

(54) **VORRICHTUNG ZUR PRÄPARATION UND ABGABE EINER ANTISEPTISCHEN WUNDSPÜLLÖSUNG**
DEVICE FOR PREPARING AND DISPENSING AN ANTISEPTIC WOUND RINSING SOLUTION
DISPOSITIF DE PRÉPARATION ET DE DISTRIBUTION DE SOLUTION ANTISEPTIQUE POUR LE LAVAGE DES PLAIES

(43) Veröffentlichungstag der Anmeldung: 06.11.2024
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- FR-A1- 2 773 735
- US-A1- 2007 249 996
- US-A1- 2011 171 283
- US-A1- 2013 289 530
- US-B2- 7 195 623
- US-B2- 7 900 659

## Beschreibung

### GEBIET DER ERFINDUNG

Die vorliegende Erfindung betrifft das Gebiet der Medizintechnik, insbesondere Vorrichtungen zur Präparation und Abgabe von Wirkstofflösungen, beispielsweise Wundspüllösungen.

### TECHNISCHER HINTERGRUND

Antiseptische Wirkstoffe und antiseptische Lösungen sind seit dem 19. Jahrhundert bekannt. Ein Beispiel für einen solchen Wirkstoff sind Hypochlorit-Salze und deren Lösungen. Die Haltbarkeit von wässrigen Hypochlorit-Lösungen ist beschränkt. Ursächlich hierfür ist hauptsächlich die langsam voranschreitende Umsetzung der Hypochlorit-Ionen zu Chlorat-Ionen. Das bedeutet, dass der Hypochlorit-Gehalt der Lösungen mit zunehmender Lagerungszeit abnimmt. Daneben ist auch ein Zerfall von Hypochlorid-Ionen zu Chlorid-Ionen und Sauerstoff möglich.

Antiseptische Hypochlorit-Lösungen sind im Stand der Technik bekannt, wobei verschiedene Lösungen zur Verbesserung der Lagerungsstabilität vorgeschlagen worden sind.

Solche Lösungen wurden beispielsweise in den nachfolgenden Veröffentlichungen beschrieben: US8945630B2, US2011/0236490A1, WO2021001789, US2019328776A1, WO2021134041A1, WO2022/038507A1, WO2020252433, WO2020174436A1, US243781, US3749672, US6162371, WO1991/003936, US6471974, US2009/0258083, WO2010148004, WO2010148004, US2009/0148342A1, WO2016/100543A2, WO2020/089693A1, und WO2021/162736A1.

Allerdings ist ein vollständiger Schutz vor Zersetzung der Hypochlorit-Ionen in wässriger Lösung weiterhin herausfordernd.

Eine weitere Herausforderung ist die Bereitstellung von solchen Lösungen in steriler Form, wobei angestrebt wird, dass auch die Behältnisse steril und stabil sein sollen. Mit dieser Aufgabe beschäftigen sich beispielsweise US4964261A und US2005232848A1.

Feste Vorstufen zur Herstellung von Hypochlorit-haltigen Lösungen sind in DE2712574A1 und US4104190A1 beschrieben.

US 2013/289530 A1 offenbart ein Gerät zur Entnahme vorgemischter Arzneimittel oder zur Rekonstitution gefriergetrockneter Arzneimittel und zur Injektion des rekonstituierten

Arzneimittels in den Patienten. Das Gerät umfasst eine Spritzenanordnung und eine Adapteranordnung, die abnehmbar mit einem Medikamentenbehälter verbunden werden kann, der ein vorgemischtes oder gefriergetrocknetes Medikament enthält. Die Spritzenanordnung des Geräts umfasst eine Flüssigkeitskammer zwischen dem vorderen Ende des Körperabschnitts und dem Kolben sowie eine Spritzenkanülenanordnung. Die Spritzenkanülenanordnung, die abnehmbar mit dem Körperabschnitt verbunden werden kann, umfasst eine Kanülenhalterung und eine Injektionsnadel, die abdichtend mit der Kanülenhalterung verbunden ist. Die Adapteranordnung umfasst einen Adapter, der vorzugsweise aus einem formbaren Kunststoff geformt ist und eine obere Wand, eine Adapterkanüle, die mit der oberen Wand verbunden ist und von dieser absteht, und eine Vielzahl von Verbindungsstücken, die mit der oberen Wand verbunden sind, um den Adapter lösbar mit dem Medikamentenbehälter zu verbinden.

US 7 900 659 B2 offenbart ein druckausgleichendes Zugangsgerät und Verfahren zum Zuführen von Fläschcheninhalten, das eine geschlossene und versiegelte Rekonstitution des Fläschcheninhalts ermöglicht. Ein starrer Behälter mit einem festen Innenvolumen ist mit einem Entlüftungslumen verbunden, das in die Phiole hineinreicht. Wenn der Druck in der Ampulle steigt, wird er durch Veränderung des Volumens eines Fachs innerhalb des starren Behälters an den Atmosphärendruck angeglichen. Das Fach ist mit einer Volumensteuervorrichtung versehen, die das Volumen des Fachs im starren Behälter automatisch variiert, um den Druck in der Ampulle durch Vergrößerung oder Verkleinerung des Fachvolumens anzupassen und auszugleichen. In einem Fall besteht die Volumenkontrollvorrichtung aus einer Gleitscheibe und in einem anderen Fall aus einer Blase, die sich bei einer Vergrößerung des Behältervolumens zusammendrückt und bei einer Verringerung des Behältervolumens ausdehnt.

FR2773735A1 offenbart eine Kammer zum Transfer einer Flüssigkeit in, oder Abgabe aus, einem Behälter. Die Kammer weist eine flexible und gegenüber dem äußeren Medium im Wesentlichen undurchlässige Wand auf und ist mit separaten Kupplungen zum Verbinden eines Behälters mit Transfer- und Abgabeeinheiten versehen. Es kann auch an ein Gefäß angeschlossen werden, das eine andere Substanz enthält, beispielsweise zum Mischen mit dem Produkt im Behälter.

US 2007/249996 A1 offenbart eine Vorrichtung mit einer Kammer innerhalb einer Spritze. Ein Flüssigkeitsdurchgang erstreckt sich durch einen Spritzenkolben. Mit dem Durchgang ist ein Ventil verbunden, das den Flüssigkeitsdurchgang durch den Kolben steuert. Die Erfindung umfasst eine Durchstichstruktur mit einem Kopfsegment und einem Körperabschnitt mit einem Kanal durch den Körperabschnitt und durch mindestens eine Oberfläche des Kopfes, ohne durch die Spitze zu verlaufen. In einem weiteren Aspekt umfasst die Erfindung ein Verfahren zur Herstellung eines Mittels zur Verabreichung an ein Individuum. Eine erste Komponente ist in einer Spritze und eine zweite Komponente in einer Ampulle enthalten. Ein geschlossenes Ventil ist mit einem Flüssigkeitsdurchgang zwischen der Phiole und dem Spritzenzylinder über einen Kolben verbunden. Durch die Neupositionierung des Ventils wird der Flüssigkeitsdurchgang ermöglicht, und durch das Gleiten des Kolbens werden die erste und die zweite Komponente verbunden. Durch wiederholtes Gleiten des Kolbens werden die Komponenten vermischt und das Arzneimittel hergestellt.

US7195623B2 offenbart ein Kit und eine Nadelanordnung zum Rekonstituieren einer Menge eines gefriergetrockneten Arzneimittels. Das Kit der Erfindung kann ein Rekonstitutionsgerät mit einem nicht transparenten Nadelabdeckungsabschnitt mit einer Aushöhlung umfassen, die dazu geeignet ist, das eingesteckte, mit Stopfen versehene Ende der Injektionspenpatrone aufzunehmen, wobei sich der Nadelabdeckungsabschnitt um eine seitlich abfeuernde Kanüle herum erstreckt, um die Kanüle vor seitlichen Blicken zu verbergen. Ein verschiebbares Ausrichtungselement, das innerhalb des Nadelabdeckabschnitts in einer ersten axialen Position gehalten wird und in eine zweite axiale Position verschiebbar ist, wenn die Injektionspenpatrone während des Einführens daran anstößt, kann enthalten sein. Das Set enthält eine Injektionspen-Patrone mit einem Zylinder, der mit einer bestimmten Menge eines gefriergetrockneten Arzneimittels gefüllt ist, einen verschiebbaren Kolben, der während des Pen-Betriebs zum Ausstoßen des rekonstituierten Arzneimittels verwendet wird, und einen Stopfen, der während der Rekonstitution des Arzneimittels von der Kanüle durchdrungen werden kann. Die Nadelanordnung der Erfindung kann eine Kanüle umfassen, die einen axialen Durchgang durch sie hindurch bildet, der mit einem Reservoir einer Verdünnungsflüssigkeit in Verbindung steht.

US 2011/171283 A1 offenbart eine Zusammensetzung enthaltend mindestens einen nutritiven, mindestens einen desinfizierenden bzw. dekontaminierenden und/oder mindestens einen proteaseinhibierenden Wirkstoff und/oder Wirkstoffkomplex zur äußerlichen Pflege und/oder Behandlung von Wunden des menschlichen oder tierischen Körpers.

### BEVORZUGTE AUSFÜHRUNGSFORMEN

Die Erfindung umfasst eine Vorrichtung zur Herstellung einer antiseptischen Wundspüllösung nach Anspruch 1, ein Kit nach Anspruch 13, ein Verfahren nach Anspruch 14 und, gemäß Anspruch 15, die Verwendung der Vorrichtung nach Anspruch 1. Eine Aufgabe der vorliegenden Erfindung besteht darin, eines oder mehrere der oben geschilderten und weitere Probleme des Stands der Technik zu lösen. Beispielsweise liefert die Erfindung eine wirkstoffhaltige Vorrichtung, mit dessen Hilfe sich eine gebrauchsfertige Wirkstofflösung mithilfe handelsüblicher Behälter mit steriler Kochsalzlösung oder vergleichbaren Lösungen schnell, einfach und kostengünstig herstellen lässt. Da die

erfindungsgemäße Vorrichtung den Wirkstoff in fester Form enthält, kann die Vorrichtung längere Zeit gelagert werden, auch wenn sie einen Wirkstoff enthält, der in wässriger Lösung nicht dauerhaft stabil ist. Zudem entfällt bei der Herstellung der erfindungsgemäßen Vorrichtung die Sterilisation von Flüssigkeiten. Einige Wirkstoffe lassen sich in fester Form besser sterilisieren, da sie sich in Lösung während der Sterilisierung leichter zersetzen. In einigen Ausführungsformen liefert die erfindungsgemäße Vorrichtung die Möglichkeit, die darin hergestellte Flüssigkeit direkt an einen Patienten abzugeben, beispielsweise mithilfe einer Düse, die an der erfindungsgemäßen Vorrichtung angebracht werden kann.

Diese Aufgaben werden gelöst durch die hierin beschriebenen Verfahren und Vorrichtungen, insbesondere denjenigen, die in den Patentansprüchen beschrieben sind.

Bevorzugte Ausführungsformen der Erfindung werden nachstehend beschrieben.

Eine erste Ausführungsform betrifft eine Vorrichtung zur Herstellung einer antiseptischen Wundspüllösung, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist,
- ein Reservoir, das einen Wirkstoff in fester Form enthält, und über einen Ausgang des Reservoirs fluidleitend mit dem Anschlusselement verbindbar ist;
- einen flüssigkeitsdurchlässigen Filter, der an dem Ausgang des Reservoirs angeordnet ist, und eingerichtet ist, den Wirkstoff in fester Form in dem Reservoir zurückzuhalten;
- ein Fluidikelement, welches zur Aufnahme einer Flüssigkeit aus einem Behälter in das Reservoir eingerichtet ist, und/oder zur Abgabe einer Flüssigkeit aus dem Reservoir eingerichtet ist;
wobei die Vorrichtung ausgebildet und eingerichtet ist, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben, wobei der Wirkstoff ein Antiseptikum ist, welches ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat.

Eine zweite Ausführungsform betrifft eine Vorrichtung nach Ausführungsform 1, wobei der Dorn einen hohlen Innenraum aufweist, der zur Aufnahme einer Flüssigkeit aus einem Behälter in das Reservoir eingerichtet ist.

Eine dritte Ausführungsform betrifft eine Vorrichtung nach einer der vorangehenden Ausführungsformen, wobei das Fluidikelement einen innerhalb des Reservoirs beweglich angeordneten Kolben aufweist, der das Reservoir flüssigkeitsdicht abschließt.

Eine vierte Ausführungsform betrifft eine Vorrichtung nach einer der vorangehenden Ausführungsformen, welche ein erstes Bauteil und ein zweites Bauteil aufweist, welche lösbar miteinander verbindbar sind.

Eine fünfte Ausführungsform betrifft eine Vorrichtung nach Ausführungsform 4, wobei das erste Bauteil das Anschlusselement und den Dorn enthält, und/oder wobei das zweite Bauteil das Reservoir, den Filter und das Fluidikelement enthält.

Eine sechste Ausführungsform betrifft eine Vorrichtung nach Ausführungsform 4 oder 5, wobei die Vorrichtung weiterhin ein Gewinde aufweist, mit dessen Hilfe das erste Bauteil mit dem zweiten Bauteil verbindbar ist.

Eine siebte Ausführungsform betrifft eine Vorrichtung nach einer der vorangehenden Ausführungsformen, wobei die Vorrichtung vollständig sterilisiert ist, bevorzugt vollständig pyrogenfrei ist.

Eine achte Ausführungsform betrifft eine Vorrichtung nach einer der vorangehenden Ausführungsformen, wobei die Vorrichtung weiterhin eine Spritzdüse aufweist, welche zur Abgabe des Wirkstoffs in gelöster Form eingerichtet ist.

Eine neunte Ausführungsform betrifft eine Vorrichtung gemäß Ausführungsform 8, wobei die Spritzdüse lösbar fluidleitend mit dem Anschlusselement verbindbar ist.

Eine weitere Ausführungsform betrifft eine Vorrichtung nach einer der vorangehenden Ausführungsformen, wobei das Anschlusselement eine erste Durchführung aufweist, welche zur Aufnahme einer Flüssigkeit aus einem Behälter in das Reservoir eingerichtet ist.

Eine weitere Ausführungsform betrifft eine Vorrichtung nach einer der vorangehenden Ausführungsformen, wobei das Anschlusselement eine zweite Durchführung aufweist, welche zur Abgabe einer Flüssigkeit aus einem Behälter eingerichtet ist, wobei die Abgabe bevorzugt in einem Zustand möglich ist, in welchem die Vorrichtung über die erste Durchführung gemäß Ausführungsform 11 mit dem Reservoir verbunden ist.

Eine weitere Ausführungsform betrifft eine Vorrichtung nach einer der vorangehenden Ausführungsformen, wobei das Reservoir durchlässig für Gammastrahlen und/oder Elektronenstrahlen ist.

Ein weiterer Aspekt betrifft ein Kit, aufweisend eine Vorrichtung nach einem der Ausführungsformen 1 bis 13 und einen Behälter mit einer medizinisch verträglichen Flüssigkeit, wobei die Vorrichtung lösbar mit dem Behälter verbindbar ist, wobei das Kit gemeinsam keimdicht verpackt, bevorzugt vollständig sterilisiert, weiter bevorzugt vollständig pyrogenfrei ist.

Ebenfalls offenbart ist ein Wirkstoff zur Verwendung in einem medizinischen Behandlungsverfahren, wobei der Wirkstoff mithilfe einer Vorrichtung nach einem der vorangegangenen Ausführungsformen unmittelbar vor der Verabreichung an einen Patienten in einer Flüssigkeit gelöst und anschließend in gelöster Form verabreicht wird.

Ein weiterer Aspekt betrifft ein Verfahren zur Herstellung einer antiseptischen Wundspüllösung, welches die nachfolgenden Schritte umfasst,
a) Aufnahme einer Flüssigkeit aus einem Behälter mithilfe einer Vorrichtung nach einer der oben genannten Ausführungsformen in das Reservoir der Vorrichtung,
b) Mischen des Wirkstoffs innerhalb des Reservoirs mit der in Schritt a) aufgenommenen Flüssigkeit,
c) dadurch Erhalt einer antiseptischen Wundspüllösung,
d) gegebenenfalls Abgabe der antiseptischen Wundspüllösung in den Behälter.

Ein weiterer Aspekt betrifft die Verwendung einer Vorrichtung nach einer der Ausführungsformen 1 bis 13 zur Herstellung einer antiseptischen Wundspüllösung.

### KURZE BESCHREIBUNG DER ZEICHNUNGEN

**Figur 1** zeigt einen ersten Teil einer Vorrichtung, welche mit einem Behälter verbindbar ist.
**Figur 2** zeigt einen Behälter, der mit einer erfindungsgemäßen Vorrichtung verbunden werden kann.
**Figur 3** zeigt einen ersten Teil einer erfindungsgemäßen Vorrichtung, welche an einen Behälter angebracht ist.
**Figur 4** zeigt einen zweiten Teil einer erfindungsgemäßen Vorrichtung mit einem Reservoir für einen Wirkstoff und einem Fluidikelement zum Transport einer Flüssigkeit aus einem Behälter.
**Figur 5** zeigt eine erfindungsgemäße Vorrichtung, die mit einem Behälter verbunden ist.
**Figur 6** zeigt einen ersten Teil einer erfindungsgemäßen Vorrichtung, welcher ein Gewinde zur Verbindung mit einem Verschluss oder einem zweiten Teil der Vorrichtung aufweist.
**Figur 7** zeigt einen ersten Teil einer erfindungsgemäßen Vorrichtung gemäß Figur 6, der über das Gewinde mit einem zweiten Teil der Vorrichtung verbunden ist.
**Figur 8** zeigt einen ersten Teil einer erfindungsgemäßen Vorrichtung gemäß Figur 6, der über das Gewinde mit einer Düse verbunden ist.
**Figur 9** zeigt eine Vorrichtung, die mit einer Abgabevorrichtung verbunden ist.
**Figur 10** zeigt eine Vorrichtung, die mit einer Düse und einem Schild versehen ist.
**Figur 11** zeigt eine Düse mit einem Gelenk, die mit einer erfindungsgemäßen Vorrichtung verwendbar ist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt.

Eine erste Ausführungsform beschreibt eine Vorrichtung zur Herstellung einer Wirkstofflösung, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist;
- ein Reservoir, das einen Wirkstoff in fester Form enthält, und über einen Ausgang des Reservoirs fluidleitend mit dem Anschlusselement verbindbar ist;
- einen flüssigkeitsdurchlässigen Filter, der an dem Ausgang des Reservoirs angeordnet ist, und eingerichtet ist, den Wirkstoff in fester Form in dem Reservoir zurückzuhalten;
- ein Fluidikelement, welches zur Aufnahme einer Flüssigkeit aus einem Behälter in das Reservoir eingerichtet ist, und/oder zur Abgabe einer Flüssigkeit aus dem Reservoir eingerichtet ist;
wobei die Vorrichtung ausgebildet und eingerichtet ist, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben wobei der Wirkstoff ein Antiseptikum ist, welches ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat.

Die Vorrichtung umfasst einen Wirkstoff. Der Wirkstoff ist erfindungsgemäss ein antiseptischer Wirkstoff.

Die Wirkstofflösung ist daher eine antiseptische Wundspüllösung. Die Begriffe "antiseptisch" und "antimikrobiell" werden hierin synonym verwendet, und bezeichnen eine bakterizide oder fungizide, d. h. keimtötende, Wirkung eines Wirkstoffs. Die Vorrichtung kann weiterhin einen Hilfsstoff enthalten. Der Wirkstoff kann als Gemisch mit einem solchen Hilfsstoff vorliegen. Der Hilfsstoff kann beispielsweise eine Puffersubstanz umfassen. In einer Ausführungsform ist der Hilfsstoff ausgewählt aus der Gruppe bestehend aus Glucuronsäure, Galacturonsäure, Gluconsäurelacton, Natriumgluconat, Natriumhydrogensulfat, Natriumdihydrogenphosphat, Calciumdihydrogenphosphat, Citronensäure und Mononatriumcitrat.

Die Vorrichtung weist ein Anschlusselement auf. Das Anschlusselement ist zum flüssigkeitsdichten Anschluss der Vorrichtung an einen Behälter ausgebildet und eingerichtet. Solche Behälter können beispielsweise Verpackungen sein, in denen physiologische Kochsalzlösungen, Infusionslösungen und ähnliche flüssige Medizinprodukte handelsüblich erhältlich sind. Gebräuchlich hierfür sind beispielsweise Flaschen oder Folienbeutel. Diese Behälter bestehen häufig aus Kunststoff. Der Behälter kann ein Behälter mit flexibler, bevorzugt elastisch verformbarer, Außenwand sein, sodass eine darin enthaltene Flüssigkeit durch Zusammendrücken des Behälters aus dem Behälter freigesetzt werden kann. Der Behälter kann ein Septum umfassen, welches mit dem Dorn der Vorrichtung durchstochen werden kann. Das Anschlusselement der Vorrichtung kann bevorzugt formschlüssig mit einem Behälter verbindbar sein. Beispielsweise kann das Anschlusselement an die Öffnung einer Kunststoffflasche oder den Anschluss eines Folienbeutels aufsteckbar sein.

Die Vorrichtung enthält weiterhin einen Dorn, der zum Durchstechen eines Septums eines Behälters ausgebildet und eingerichtet ist. Der Dorn kann hierzu eine spitz zulaufende Form aufweisen, mit dessen Spitze ein Septum eines Behälters durch manuelles Ausüben von Druck des Dorns auf das Septum durchdrungen werden kann. Vergleichbare Strukturen werden zum Anschluss von Infusionbestecken an Behälter mit Infusionslösungen verwendet.

Die Vorrichtung weist weiterhin ein Reservoir auf. Das Reservoir enthält einen Wirkstoff in fester Form, beispielsweise als Pulver. Das Reservoir kann eine im wesentlichen zylindrische Form aufweisen, ähnlich dem Innenraum einer Spritze.

Die Vorrichtung kann eingerichtet sein, den enthaltenen Wirkstoff in gelöster Form in den Behälter abzugeben.

An einem Ausgang des Reservoirs ist ein Filter angeordnet. Dieser Filter ist eingerichtet, den Wirkstoff in dem Reservoir zurückzuhalten, solange der Wirkstoff nicht in einer Flüssigkeit gelöst ist. Auf diese Weise kann verhindert werden, dass Wirkstoff in ungelöster Form an einen Patienten abgegeben wird. Erwünscht ist hingegen, dass der Wirkstoff erst in ausschließlich gelöster Form an einen Patienten abgegeben werden kann. Hierzu kann der Filter bevorzugt flüssigkeitsdurchlässig sein. Der Filter ist bevorzugt gasdurchlässig. Ein gasdurchlässiger Filter ermöglicht beispielsweise eine leichtere Befüllung des Reservoirs mit Wirkstoff, insbesondere bei der Herstellung der erfindungsgemäßen Vorrichtung. Hierzu kann der Wirkstoff bevorzugt mithilfe von Druckluft oder Vakuum in das Reservoir eingebracht werden, wobei der gasdurchlässige Filter den Durchtritt des Gases ermöglicht, aber den Wirkstoff im Reservoir zurückhält.

Der Filter kann beispielsweise die Form einer Scheibe aufweisen. Der Filter weist bevorzugt eine offene Porosität auf. In einigen Ausführungsformen weist der Filter offene Poren mit einer Porengröße kleiner 5 µm, bevorzugt kleiner 3 µm und besonders bevorzugt kleiner 1 µm auf. Diese Porengröße kann durch den Ausschluss von sphärischen Partikeln mit einem entsprechenden Teilchendurchmesser bestimmt werden. Es handelt sich daher um nominelle Porengröße.

Der Filter kann durch eine Halterung innerhalb des Reservoirs befestigt sein. Die Halterung kann beispielsweise in Form eines Klemmrings ausgebildet sein. Gegebenenfalls kann der Filter in einer Aussparung in einer Wand des Reservoirs befestigt sein.

Der Filter kann beispielsweise Polyethylen, Polyurethan, Polyimid, Polyethersulfon, Polyvinylidenfluorid oder Poltetrafluorethylen aufweisen.

Der Filter kann ein Gewebefilter (woven filter) oder ein Vliesfilter (non-woven filter) sein.

In einigen Ausführungsformen umfasst das Reservoir einen zweiten Filter, der das Reservoir in zwei voneinander getrennte Bereiche, also einen ersten Bereich und einen zweiten Bereich, unterteilt. Der zweite Filter kann durch eine zweite Halterung innerhalb des Reservoirs befestigt sein. Der erste Bereich kann einen ersten Wirkstoff enthalten, und der zweite Bereich kann einen zweiten Wirkstoff enthalten, welcher sich von dem ersten Wirkstoff unterscheidet. Der erste Wirkstoff und der zweite Wirkstoff befinden sich bevorzugt jeweils in fester Form, beispielsweise Pulverform. Eine solche Ausführungsform ermöglicht die Herstellung einer Wirkstofflösung mit zwei verschiedenen Wirkstoffen, ohne dass die beiden Wirkstoffe in fester Form während der Lagerung der Vorrichtung miteinander in Berührung kommen.

Das Reservoir kann derart dimensioniert sein, dass das Verhältnis der Volumina des Reservoirs und des Behälters in einem Verhältnis von 1:25 zu 1:500 liegt. Das Volumen des Behälters kann beispielsweise 250 ml, 500 ml oder 1000 ml betragen. Dementsprechend kann das Volumen des Reservoirs beispielsweise 0,5 bis 10 ml, 1 bis 20 ml, oder 2 bis 40 ml betragen.

Die Vorrichtung weist weiterhin ein Fluidikelement auf. Das Fluidikelement ist bevorzugt zur Aufnahme einer Flüssigkeit aus einem Behälter in das Reservoir eingerichtet. Das Fluidikelement kann alternativ oder zusätzlich zur Abgabe einer Flüssigkeit aus dem Reservoir eingerichtet sein. Das Fluidikelement ist bevorzugt zum aktiven Fördern einer Flüssigkeit eingerichtet. Das Fluidikelement kann beispielsweise einen Kolben zum Fördern einer Flüssigkeit aufweisen. Das Fluidikelement kann eine Spritze oder eine Pumpe aufweisen.

In einer Ausführungsform weist der Dorn einen hohlen Innenraum auf, der zur Aufnahme einer Flüssigkeit aus einem Behälter in das Reservoir eingerichtet ist. Hierbei kann der Dorn beispielsweise in der Form einer Hohlnadel ausgestaltet sein. Der Dorn kann beispielsweise aus Kunststoff bestehen, oder einen Kunststoff umfassen.

Bevorzugt ist der Dorn ausreichend steif, um ein Septum eines handelsüblichen Behälters für physiologische Kochsalzlösung zu durchdringen, jedoch nicht so hart und fest, dass eine Verletzungsgefahr für medizinisches Personal besteht. In einer Ausführungsform ist der Dorn keine Metallnadel.

In einer Ausführungsform weist das Fluidikelement einen innerhalb des Reservoirs beweglich angeordneten Kolben auf, der das Reservoir flüssigkeitsdicht abschließt. Das Fluidikelement kann somit beispielsweise als Spritze oder Spritzenpumpe ausgeführt sein. Das Fluidikelement kann auch andere Arten von Mitteln zur Bewegung einer Flüssigkeit aufweisen, z.B. eine Membranpumpe, Peristaltikpumpe, Druckluftkartusche, oder ein Anschluss für eine externe Druck- oder Vakuumquelle.

In einer Ausführungsform weist die Vorrichtung ein erstes Bauteil und ein zweites Bauteil auf, welche lösbar miteinander verbindbar sind. Beispielsweise kann das erste Bauteil das Anschlusselement und den Dorn enthalten, und/oder das zweite Bauteil kann das Reservoir, den Filter und das Fluidikelement enthalten. Das erste Bauteil und das zweite Bauteil können durch ein Verbindungselement, bevorzugt ein formschlüssiges Verbindungselement, beispielsweise mithilfe eines Gewindes oder Schnapphakens, miteinander verbindbar sein. Ein bevorzugtes Gewinde ist ein Luer-Lock-Gewinde. Das erste Bauteil kann bevorzugt ein Außengewinde aufweisen, das mit einem Innengewinde des zweiten Bauteils verbindbar ist. Das zweite Bauteil kann ein Außengewinde aufweisen, das mit einem Innengewinde des ersten Bauteils verbindbar ist.

Das erste Bauteil kann in einer Ausführungsform dazu eingerichtet sein, fluidleitend mit einem Behälter verbunden zu sein, während das zweite Bauteil vom ersten Bauteil gelöst und gegebenenfalls gegen eine andere Vorrichtung ausgewechselt wird, beispielsweise gegen einen Verschluss oder eine Düse. Das erste Bauteil kann somit als eine Art multifunktionaler Schnellverbinder für andere Komponenten dienen. Das zweite Bauteil kann eine Spritze umfassen, sonders bevorzugt eine Luer-Lock-Spritze.

Wie oben dargestellt kann die Vorrichtung ein Verbindungselement umfassen, um entweder einzelne Teile der erfindungsgemäßen Vorrichtung, insbesondere das erste Bauteil und das zweite Bauteil, miteinander zu verbinden, oder die Vorrichtung mit weiteren Vorrichtungen zu verbinden, oder beides. Hierbei kann entweder ein einziges Verbindungselement dazu eingerichtet sein, alternativ mit einer Vielzahl unterschiedlicher Teile verbunden zu werden, oder es können mehrere Verbindungselemente an unterschiedlichen Positionen der Vorrichtung vorgesehen sein. Bevorzugt ist ein Verbindungselement ein formschlüssiges Verbindungselement. Ein Beispiel für ein solches Verbindungselement ist ein Gewinde. In einigen Ausführungsformen ist das Verbindungselement ein Luer-Lock-Gewinde.

In einer Ausführungsform weist die Vorrichtung ein Verbindungselement auf, mit dessen Hilfe das erste Bauteil mit dem zweiten Bauteil verbindbar ist. In einigen Ausführungsformen ist dasselbe Verbindungselement zusätzlich eingerichtet, die Vorrichtung mit einem Verschluss, einer Düse oder einer sonstigen Abgabevorrichtung zu verbinden. Alternativ können jeweils einzelne, voneinander verschiedenen Verbindungselemente für diese Zwecke vorgesehen sein.

In einer Ausführungsform ist die Vorrichtung vollständig sterilisiert. In einer Ausführungsform ist die Vorrichtung vollständig pyrogenfrei. Der Begriff "sterilisiert" bezieht sich darauf, dass die Vorrichtung einem Verfahren unterzogen worden ist, um Krankheitserreger zu entfernen oder abzutöten, sodass die Vorrichtung gemäß der üblichen Standards wie zum Beispiel der Norm DIN EN 556-1 medizinisch verwendet werden kann. Verfahren zur Sterilisation umfassen beispielsweise Autoklavierung, Bestrahlung mit Elektronenstrahlen oder Gammastrahlen, oder Behandlung mit desinfizierenden Substanzen wie zum Beispiel Ethylenoxid. In ähnlicher Weise bezieht sich der Begriff "pyrogenfrei" darauf, dass die Vorrichtung einer im Fachgebiet üblichen Behandlung unterzogen worden ist, um Pyrogene zu entfernen oder zu zersetzen. Ob die Vorrichtung pyrogenfrei ist, kann mithilfe des Limulus-Amöbozytenlysat (LAL)-Tests gemäß Ph. Eur. Kapitel 2.6.14 geprüft werden. Hierbei gelten jeweils die Normen bzw. Dokumente, die zum Prioritätstag der vorliegenden Anmeldung aktuell sind.

In einer Ausführungsform weist die Vorrichtung weiterhin eine Düse auf, welche zur Abgabe des Wirkstoffs in gelöster Form eingerichtet ist. Die Düse kann einen im Wesentlichen zylindrischen, an einem Ende spitz zulaufenden Hohlkörper aufweisen. Die Düse kann gegebenenfalls ein Gelenk umfassen, das durch seitliches Biegen einer Öffnung der Düse relativ zur gesamten Vorrichtung die Abgabe einer Flüssigkeit in unterschiedliche Richtungen erlaubt.

Bevorzugt ist die Düse lösbar fluidleitend mit dem Anschlusselement verbindbar. Dies kann über ein formschlüssiges Verbindungselement, beispielsweise ein Gewinde, bevorzugt ein Luer-Lock-Gewinde, ermöglicht sein.

Die Vorrichtung kann weiterhin einen Schild umfassen. Der Schild kann eingerichtet sein, Teile der Vorrichtung vor Spritzern der abgegebenen Flüssigkeit, insbesondere einer Wirkstofflösung, zu schützen. Der Schild kann an oder in räumlicher Nähe zu der Düse angeordnet sein. Der Schild kann die Form einer Scheibe aufweisen. Der Schild kann eine konkave Krümmung aufweisen, ähnlich einer Parabolantenne. Der Schild kann eine zentral angeordnete Ausnehmung aufweisen, die zur Anbringung des Schilds an einem Ausgang der Vorrichtung eingerichtet ist.

Erfindungsgemäß ist der in der Vorrichtung enthaltene Wirkstoff ein Antiseptikum, das ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat. In einer Ausführungsform ist das Antiseptikum ein Hypochloritsalz.

In einer Ausführungsform liegt der Wirkstoff als Pulver mit einer mittleren Partikelgröße kleiner 300 µm, bevorzugt kleiner 200 µm und besonders bevorzugt kleiner 100 µm vor. Durch die kleine Partikelgröße kann eine schnelle Auflösung der Wirkstoffpartikel erzielt werden. Die Partikelgröße des Wirkstoffs kann bevorzugt oberhalb des Ausschlussvolumens des Filters liegen. Dieser mittlere Durchmesser der Partikel kann durch fraktionierte Siebung bestimmt werden, wie es im Fachgebiet üblich ist. In einer Ausführungsform liegt der Wirkstoff als zylinderförmiger Pressling oder als zylinderförmige erstarrte Schmelze vor.

In einer Ausführungsform weist das Anschlusselement eine erste Durchführung auf, welche zur Aufnahme einer Flüssigkeit aus einem Behälter in das Reservoir eingerichtet ist. Die erste Durchführung bildet bevorzugt eine fluidleitende Verbindung zwischen dem Anschlusselement, insbesondere dem Dorn, und dem Reservoir.

In einer Ausführungsform ist die Vorrichtung zur Abgabe einer Flüssigkeit aus einem Behälter eingerichtet. Hierbei ist bevorzugt die Abgabe in einem Zustand möglich, in welchem die Vorrichtung über die oben beschriebene erste Durchführung mit dem Reservoir fluidleitend verbunden ist. Beispielsweise kann mithilfe einer solchen Ausführungsform Flüssigkeit aus einem mit der Vorrichtung verbundenen Behälter zunächst in das Reservoir der Vorrichtung aufgenommen werden, der enthaltene Wirkstoff in der Flüssigkeit gelöst werden, und der gelöste Wirkstoff nachfolgend in den Behälter abgegeben werden, während die Vorrichtung mit dem Behälter verbunden ist. Anschließend kann der gelöste Wirkstoff über die Vorrichtung aus dem Behälter an einen Patienten abgegeben werden, während die Vorrichtung, insbesondere das erste Bauteil der Vorrichtung, weiterhin mit dem Behälter verbunden ist. Gegebenenfalls kann die Vorrichtung zur Abgabe des gelösten Wirkstoffs mit einer Düse oder einer anderen Abgabevorrichtung verbunden werden, wobei ggf. das zweite Bauteil der Vorrichtung gegen die Düse oder andere Abgabevorrichtung ausgewechselt werden kann. Die Flüssigkeit ist bevorzugt eine medizinisch verträgliche Flüssigkeit. Die Flüssigkeit ist bevorzugt zur inneren Anwendung geeignet.

In einer Ausführungsform ist das Reservoir durchlässig für Gammastrahlen und/oder Elektronenstrahlen. Hierdurch wird es ermöglicht, den Innenraum und den enthaltenen Wirkstoff der Vorrichtung mithilfe von Gammastrahlen und/oder Elektronenstrahlen zu sterilisieren, um eine vollständig sterilisierte Vorrichtung zu erhalten.

In einer Ausführungsform definieren der Dorn und das Reservoir gemeinsam eine Transferachse, wobei entlang dieser Transferachse eine Abgabe der Flüssigkeit aus dem Reservoir und/oder in das Reservoir erfolgen kann. In einer Ausführungsform definieren der Behälter und das Reservoir gemeinsam eine Transferachse, wobei entlang dieser Transferachse eine Abgabe der Flüssigkeit aus dem Reservoir und/oder eine Aufnahme in das Reservoir erfolgen kann.

Ein weiterer Aspekt betrifft ein Kit, aufweisend eine Vorrichtung gemäß einer der vorangehenden Ausführungsformen und einen Behälter mit einer medizinisch verträglichen Flüssigkeit, wobei die Vorrichtung lösbar mit dem Behälter verbindbar ist. In einer Ausführungsform sind die Bestandteile des Kits, nämlich die Vorrichtung und der Behälter, gemeinsam keimdicht verpackt. In einer Ausführungsform sind die Bestandteile des Kits jeweils vollständig sterilisiert. In einer Ausführungsform sind die Bestandteile des Kits jeweils vollständig pyrogenfrei. Das Kit kann weiterhin eine Abgabevorrichtung zur Abgabe einer Flüssigkeit enthalten, beispielsweise eine Düse oder Spülvorrichtung, beispielsweise eine Spülvorrichtung gemäß EP4035605A1, welche hiermit durch Bezugnahme vollständig mit aufgenommen ist. Die medizinisch verträgliche Flüssigkeit kann eine Infusionslösung oder medizinische Spüllösung umfassen. Die medizinisch verträgliche Flüssigkeit kann eine physiologische Kochsalzlösung, eine Ringer-Lösung, oder eine vergleichbare kochsalzhaltige Lösung umfassen. Die medizinisch verträgliche Flüssigkeit ist bevorzugt steril und/oder pyrogenfrei.

Weiterhin offenbart ist ein Wirkstoff zur Verwendung in einem medizinischen Behandlungsverfahren, wobei der Wirkstoff mithilfe einer hierin beschriebenen Vorrichtung unmittelbar vor der Verabreichung an einen Patienten in einer Flüssigkeit gelöst und anschließend in gelöster Form verabreicht wird. Der Wirkstoff kann ein Stoff sein, welcher eine pharmakologische Wirkung aufweist. Der Wirkstoff kann beispielsweise eine antimikrobielle Wirkung aufweisen.

Weiterhin offenbart ist ein medizinisches Behandlungsverfahren, wobei der Wirkstoff mithilfe einer hierin beschriebenen Vorrichtung unmittelbar vor der Verabreichung an einen Patienten wie hierin beschrieben in einer Flüssigkeit gelöst und anschließend in gelöster Form verabreicht wird. Die Flüssigkeit ist bevorzugt eine medizinisch verträgliche Flüssigkeit wie hierin beschrieben.

Ein weiterer Aspekt betrifft ein Verfahren zur Herstellung einer Wirkstofflösung, welches die nachfolgenden Schritte umfasst,
a) Aufnahme einer Flüssigkeit aus einem Behälter mithilfe einer hierin beschriebenen Vorrichtung in das Reservoir der Vorrichtung,
b) Mischen des Wirkstoffs innerhalb des Reservoirs der Vorrichtung mit der in Schritt a) aufgenommenen Flüssigkeit,
c) dadurch Erhalt einer Wirkstofflösung,
d) gegebenenfalls Abgabe der Wirkstofflösung in den Behälter.

Das Verfahren kann bevorzugt in der Reihenfolge a), b), c) oder a), b), c), d) durchgeführt werden. Das Verfahren kann weiterhin einen Schritt umfassen, in welchem die Wirkstofflösung aus der Vorrichtung oder aus dem Behälter abgegeben wird. Bevorzugt kann die Wirkstofflösung aus dem Behälter über die Vorrichtung abgegeben werden, wie hierin andernorts ausführlicher beschrieben.

Weiterhin offenbart ist eine Wirkstofflösung, die nach einem oben dargestellten Verfahren herstellbar ist.

Ein weiterer Aspekt betrifft eine Verwendung einer hierin beschriebenen Vorrichtung oder eines hierin beschriebenen Kits zur Herstellung einer Wirkstofflösung.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

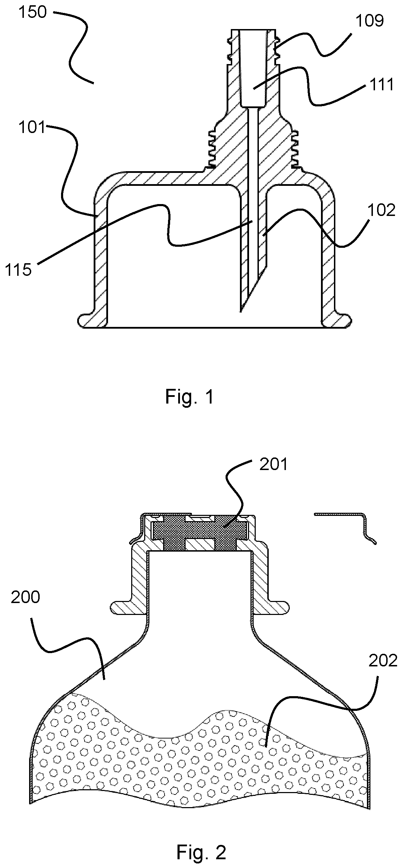

**Figur 1** zeigt beispielhaft ein erstes Bauteil **150** einer Vorrichtung **100,** welches mit einem Behälter **200** verbindbar ist. Das erste Bauteil **150** der Vorrichtung weist einen Anschlusselement **101** auf, welches formschlüssig mit einem Behälter verbunden werden kann, beispielsweise mit einem dafür vorgesehenen Anschluss am Kopf einer Infusionsflasche oder eines Folienbeutels. Die Vorrichtung weist weiterhin einen Dorn **102** auf, mit dem ein Septum **201** eines Behälters durchstochen werden kann. Der Dorn **102** weist einen inneren Hohlraum **115** auf, um eine Flüssigkeit aus dem Behälter in die Vorrichtung zu leiten. Der innere Hohlraum ist mit einer Durchführung **111** der Vorrichtung fluidleitend verbunden. Das erste Bauteil **150** der Vorrichtung **100** weist weiterhin ein Verbindungselement **109** auf, mit dem das erste Bauteil **150** der Vorrichtung mit einem zweiten Bauteil **160** der Vorrichtung verbindbar ist. Das Verbindungselement **109** ist in diesem Beispiel als Luer-Lock-Gewinde ausgeführt.

**Figur 2** zeigt einen Behälter **200,** der mit einer erfindungsgemäßen Vorrichtung verbunden werden kann. Der Behälter **200** enthält eine medizinisch verträgliche Flüssigkeit **202,** und weist ein Septum **201** auf, welches den Behälter nach außen abdichtet. Das Septum **201** ist aus einem elastischen Material, beispielsweise Silikon, gebildet. Das Septum kann zusätzlich durch ein Siegel geschützt sein, das vor der Verbindung mit der erfindungsgemäßen Vorrichtung entfernt werden kann.

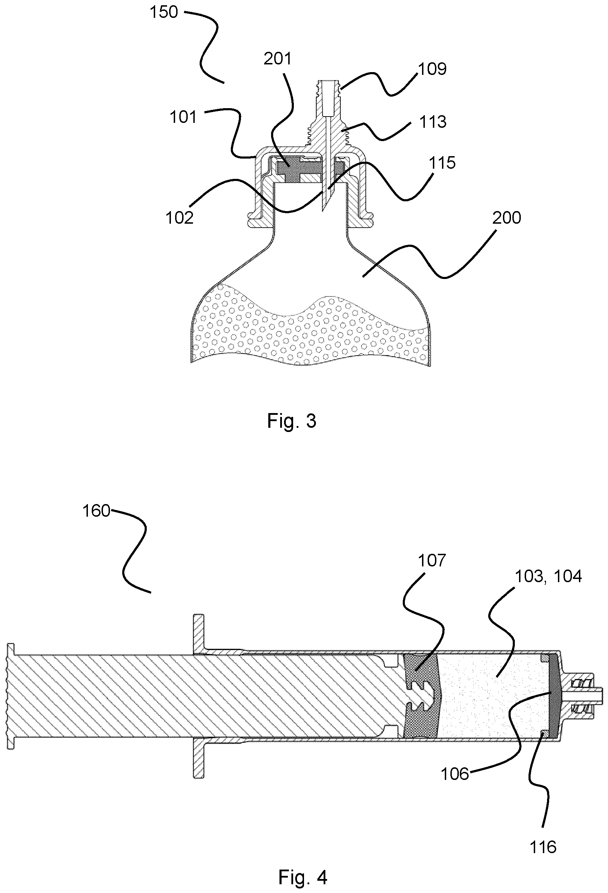

**Figur 3** zeigt ein erstes Bauteil **150** einer erfindungsgemäßen Vorrichtung, welche an einen Behälter **200** angebracht ist. Der Dorn **102** durchdringt das Septum **201** des Behälters **200,** sodass die Flüssigkeit **202** aus dem Behälter durch den Hohlraum **115** und die erste Durchführung **109** in die Vorrichtung überführt werden kann. Die Vorrichtung umfasst ein erstes Gewinde **109** und ein zweites Gewinde **113.** Beide Gewinde **(109, 113)** des ersten Bauteils **150** sind hier als Außengewinde ausgestaltet.

**Figur 4** zeigt einen zweiten Teil **160** einer erfindungsgemäßen Vorrichtung **100** mit einem Reservoir **103** für einen Wirkstoff **104** und einem Fluidikelement **107** zum Transport einer Flüssigkeit **202** aus einem Behälter **200.** Das Fluidikelement **107** ist hier als Spritze ausgeführt. Der Wirkstoff **104** ist als festes Pulver in dem Reservoir **103** angeordnet. Die Vorrichtung umfasst weiterhin einen Filter **106,** um den Wirkstoff **104** in dem Reservoir **103** zu halten. Der Wirkstoff **104** kann den Filter **106** nur in gelöster Form passieren. Der Filter **106** ist durch eine Halterung **116** in dem Reservoir **103** befestigt, um das Reservoir **103** nach außen abzudichten, sodass kein fester Wirkstoff **104** durch den Filter **106** aus dem Reservoir **103** gelangen kann. Das zweite Bauteil **160** weist ein Verbindungselement zum Anschluss an das erste Bauteil **150** auf. Dieses Verbindungselement des zweiten Bauteils **160** ist komplementär zu dem Verbindungselement des ersten Bauteils **150** ausgestaltet. Im hier gezeigten Beispiel weist das zweite Bauteil **160** ein Luer-Lock-Innengewinde auf.

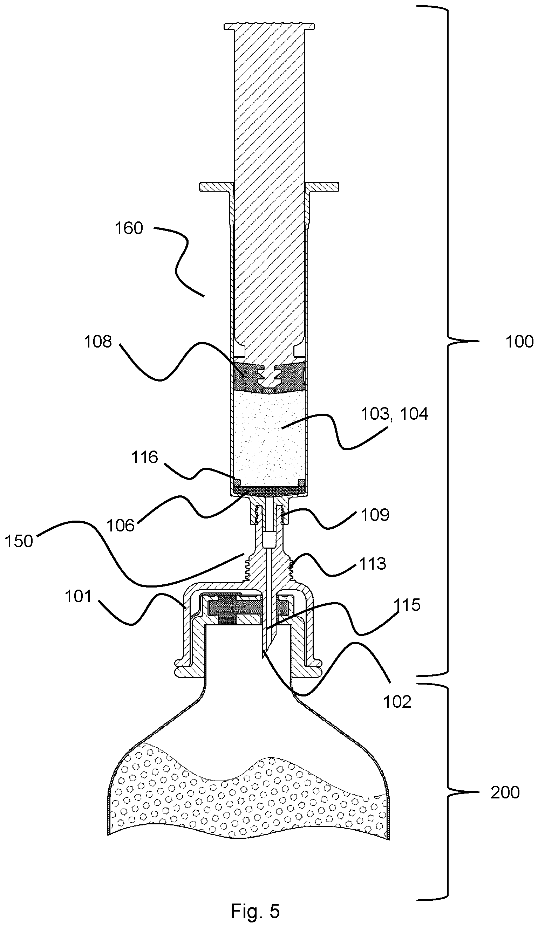

**Figur 5** zeigt eine erfindungsgemäße Vorrichtung **100,** die mit einem Behälter **200** verbunden ist. Das erste Bauteil **150** der Vorrichtung ist über ein Verbindungselement **109** mit dem zweiten Bauteil **160** der Vorrichtung fluidleitend und flüssigkeitsdicht verbunden. Das Anschlusselement **101** ist mit einem Behälter **200** verbunden, wobei der Dorn **102** durch ein Septum des Behälters in den Behälter eingeführt ist. Der Innenraum des Behälters ist durch einen Hohlraum **115** des Dorns mit dem Reservoir **103** fluidleitend verbunden. Mithilfe eines Fluidikelement, das hier einen Kolben **108** aufweist, kann nun eine Flüssigkeit aus dem Behälter **200** in das Reservoir **103** geleitet werden.

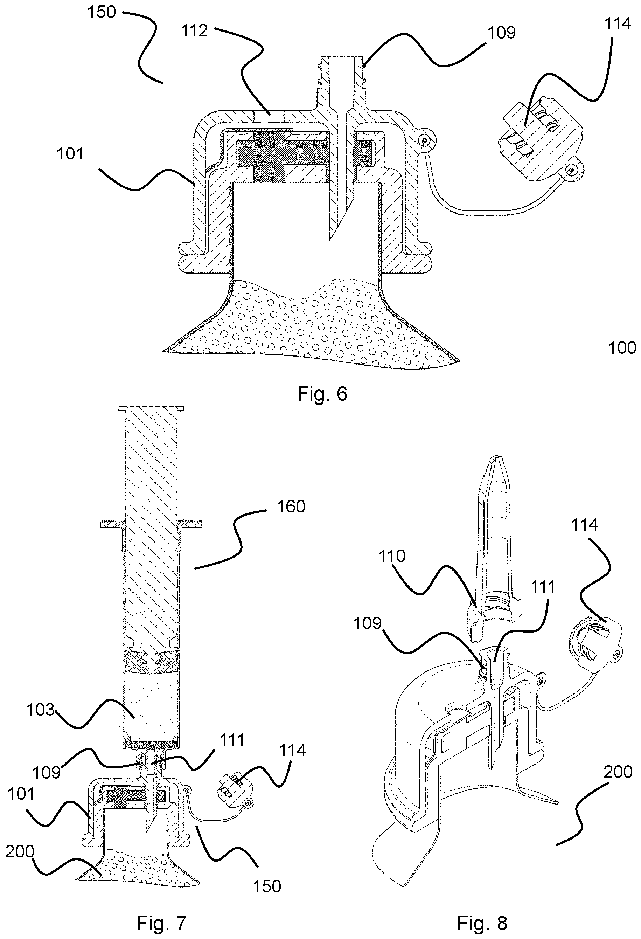

**Figur 6** zeigt ein erstes Bauteil **150** einer erfindungsgemäßen Vorrichtung, welches ein Verbindungselement **109** zur Verbindung mit einem Verschluss **114** oder einem zweiten Bauteil der Vorrichtung **160** aufweist. In der hier gezeigten Ausführungsform ist das als Gewinde ausgeführte Verbindungselement **109** alternativ mit verschiedenen Elementen verbindbar. Das Gewinde **109** kann entweder mit dem zweiten Bauteil der Vorrichtung **160** fluidleitend verbunden werden, oder mit einem Verschluss **114** flüssigkeitsdicht verschlossen werden.

**Figur 7** zeigt ein erstes Bauteil **150** einer erfindungsgemäßen Vorrichtung gemäß Figur 6, der über das Gewinde **109** mit einem zweiten Bauteil **160** der Vorrichtung verbunden ist. In dieser Konfiguration kann durch Zusammenwirken der beiden Bauteile **150** und **160** der Vorrichtung Flüssigkeit aus dem Behälter **200** entnommen werden. Die Flüssigkeit kann aus dem Behälter **200** über eine erste Durchführung **111** in das Reservoir **103** gepumpt werden. Innerhalb des Reservoirs **103** kann mithilfe der Flüssigkeit der im Reservoir **103** enthaltene Wirkstoff **104** gelöst werden, und die somit erhaltene Wirkstofflösung durch den Filter **106** in den Behälter **200** geleitet werden. Danach kann das zweite Bauteil 160 der Vorrichtung entfernt werden, und die Wirkstofflösung kann mithilfe des ersten Bauteils **150** der Vorrichtung aus dem Behälter abgegeben werden, beispielsweise an einen Patienten abgegeben werden.

**Figur 8** zeigt ein erstes Bauteil **150** einer erfindungsgemäßen Vorrichtung gemäß Figur 6, der über ein Gewinde **109** mit einer Düse **110** verbindbar ist. Mithilfe der Düse **110** und der Durchführung **111** kann die mithilfe der Vorrichtung hergestellte Wirkstofflösung aus dem Behälter **200** abgegeben werden. Gegebenenfalls kann die Durchführung **111** durch Anbringen eines Verschlusses **114** an dem Verbindungselement **109** flüssigkeitsdicht verschlossen werden.

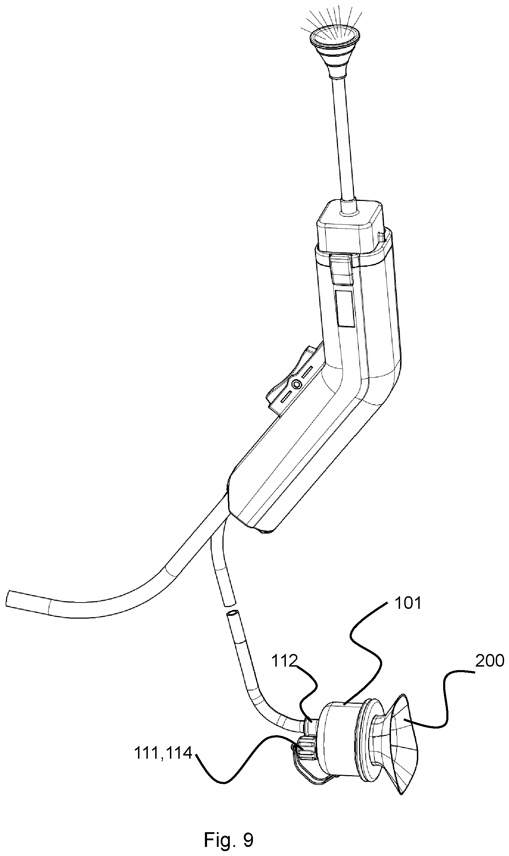

**Figur 9** zeigt eine erfindungsgemäße Vorrichtung, die gleichzeitig mit einem Behälter **200** und mit einer externen Abgabevorrichtung verbunden ist. Hierzu umfasst die Vorrichtung neben einer ersten Durchführung **111,** die hier durch einen Verschluss **114** flüssigkeitsdicht verschlossen ist, eine zweite Durchführung **112,** die zum Anschluss an eine externe Abgabevorrichtung eingerichtet ist. In der hier gezeigten Konfiguration ist das erste Bauteil **150** mithilfe des Anschlusselements **101** mit einem Behälter **200** fluidleitend verbunden, sodass eine mithilfe der Vorrichtung hergestellte Wirkstofflösung aus dem Behälter **200** über die zweite Durchführung **112** abgegeben werden kann.

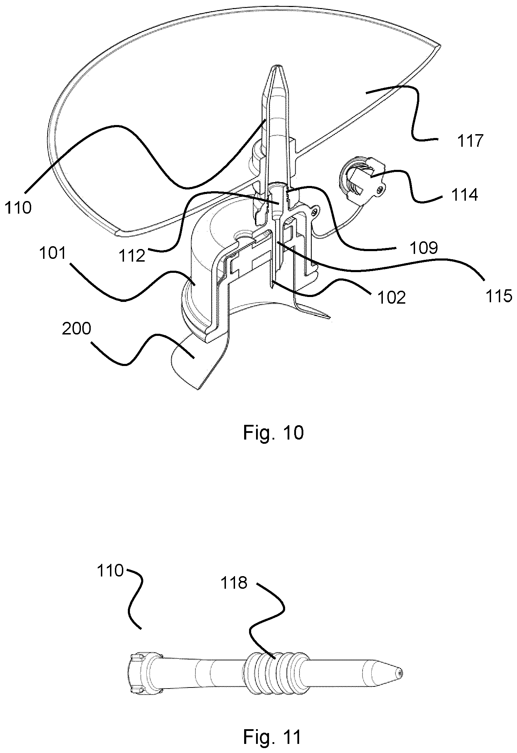

**Figur 10** zeigt ein erstes Bauteil einer Vorrichtung, die mit einer Düse **110** und einem Schild **117** versehen ist. Das erste Bauteil ist zur Abgabe einer Wirkstofflösung aus dem Behälter **200** eingerichtet. Das Schild **117** besitzt im hier gezeigten Beispiel eine zentrale, zylinderförmige Ausnehmung zur Befestigung an dem ersten Bauteil **150** und/oder der Düse **110.** Das Schild kann als Spritzschutz dienen, wenn eine Wirkstofflösung mithilfe der Düse **110** abgegeben wird.

**Figur 11** zeigt eine Düse **110** mit einem Gelenk **118,** die mit einer erfindungsgemäßen Vorrichtung verwendbar ist. Mithilfe des Gelenks **118** kann die Düse **110** gebogen werden, sodass die Austrittsöffnung der Düse einen Winkel mit dem Anschluss der Düse bildet, der sich von 180° unterscheidet. Somit kann eine Wirkstofflösung in verschiedene Richtungen abgegeben werden, ohne die gesamte Vorrichtung zu drehen.

### BEZUGSZEICHENLISTE

- 100: Vorrichtung
- 101: Anschlusselement
- 102: Dorn
- 103: Reservoir
- 104: Wirkstoff
- 105: Ausgang des Reservoirs
- 106: Filter
- 107: Fluidikelement
- 108: Kolben
- 109: erstes Verbindungselement (zur Verbindung der Bauteile)
- 110: Düse
- 111: erste Durchführung (zur Aufnahme aus Behälter)
- 112: zweite Durchführung (zum Anschluss an Pumpe o.ä.)
- 113: zweites Verbindungselement (für Düse)
- 114: Verschluss
- 115: Hohlraum des Dorns
- 116: Halterung für Filter
- 117: Schild
- 118: Gelenk
- 150: erstes Bauteil
- 160: zweites Bauteil
- 200: Behälter
- 201: Septum
- 202: Flüssigkeit

## Patentansprüche

1. Vorrichtung (100) zur Herstellung einer antiseptischen Wundspüllösung, aufweisend
- ein Anschlusselement (101), das zum flüssigkeitsdichten Anschluss an einen Behälter (200) ausgebildet und eingerichtet ist;
- einen Dorn (102), der zum Durchstechen eines Septums (201) eines Behälters ausgebildet und eingerichtet ist.
- ein Reservoir (103), das einen Wirkstoff (104) in fester Form enthält, und über einen Ausgang (105) des Reservoirs fluidleitend mit dem Anschlusselement (101) verbindbar ist;
- einen flüssigkeitsdurchlässigen Filter (106), der an dem Ausgang (105) des Reservoirs angeordnet ist, und eingerichtet ist, den Wirkstoff (104) in fester Form in dem Reservoir (103) zurückzuhalten;
- ein Fluidikelement (107), welches zur Aufnahme einer Flüssigkeit (202) aus einem Behälter (200) in das Reservoir (103) eingerichtet ist, und/oder zur Abgabe einer Flüssigkeit (202) aus dem Reservoir (103) eingerichtet ist;
wobei die Vorrichtung (100) ausgebildet und eingerichtet ist, eine Flüssigkeit (202) aus einem Behälter (200) in das Reservoir aufzunehmen, den Wirkstoff (104) in der Flüssigkeit (202) zu lösen, und den gelösten Wirkstoff über das Anschlusselement (101) an einen Patienten abzugeben,
wobei der Wirkstoff ein Antiseptikum ist, welches ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat.

2. Vorrichtung nach Anspruch 1, wobei der Dorn (102) einen hohlen Innenraum (115) aufweist, der zur Aufnahme einer Flüssigkeit (202) aus einem Behälter (200) in das Reservoir (103) eingerichtet ist.

3. Vorrichtung nach einem der vorangehenden Ansprüche, wobei das Fluidikelement (107) einen innerhalb des Reservoirs (103) beweglich angeordneten Kolben (108) aufweist, der das Reservoir flüssigkeitsdicht abschließt.

4. Vorrichtung nach einem der vorangehenden Ansprüche, welche ein erstes Bauteil (150) und ein zweites Bauteil (160) aufweist, welche lösbar miteinander verbindbar sind.

5. Vorrichtung nach Anspruch 4, wobei das erste Bauteil (150) das Anschlusselement (101) und den Dorn (102) enthält, und/oder wobei das zweite Bauteil (160) das Reservoir (103), den Filter (106) und das Fluidikelement (107) enthält.

6. Vorrichtung nach Anspruch 4 oder 5, wobei die Vorrichtung weiterhin ein Verbindungselement (109) aufweist, mit dessen Hilfe das erste Bauteil (150) mit dem zweiten Bauteil (160) verbindbar ist.

7. Vorrichtung nach einem der vorangehenden Ansprüche, wobei die Vorrichtung vollständig sterilisiert ist, bevorzugt vollständig pyrogenfrei ist.

8. Vorrichtung nach einem der vorangehenden Ansprüche, wobei die Vorrichtung weiterhin eine Düse (110) aufweist, welche zur Abgabe des Wirkstoffs in gelöster Form eingerichtet ist.

9. Vorrichtung gemäß Anspruch 8, wobei die Düse (110) lösbar fluidleitend mit dem Anschlusselement (101) verbindbar ist.

10. Vorrichtung nach einem der vorangehenden Ansprüche, wobei das Anschlusselement (101) eine erste Durchführung (111) aufweist, welche zur Aufnahme einer Flüssigkeit (202) aus einem Behälter (200) in das Reservoir (103) eingerichtet ist.

11. Vorrichtung nach einem der vorangehenden Ansprüche, wobei das Anschlusselement (101) eine zweite Durchführung (112) aufweist, welche zur Abgabe einer Flüssigkeit (202) aus einem Behälter (200) eingerichtet ist, wobei die Abgabe bevorzugt in einem Zustand möglich ist, in welchem die Vorrichtung (100) über die erste Durchführung (111) gemäß Anspruch 10 mit dem Reservoir (103) verbunden ist.

12. Vorrichtung nach einem der vorangehenden Ansprüche, wobei das Reservoir (103) durchlässig für Gammastrahlen und/oder Elektronenstrahlen ist.

13. Kit, aufweisend eine Vorrichtung (100) nach einem der Ansprüche 1 bis 12 und einen Behälter (200) mit einer medizinisch verträglichen Flüssigkeit, wobei die Vorrichtung (100) lösbar mit dem Behälter (200) verbindbar ist, wobei das Kit gemeinsam keimdicht verpackt, bevorzugt vollständig sterilisiert, weiter bevorzugt vollständig pyrogenfrei ist.

14. Verfahren zur Herstellung einer antiseptischen Wundspüllösung, welches die nachfolgenden Schritte umfasst,
a) Aufnahme einer Flüssigkeit (202) aus einem Behälter (200) mithilfe einer Vorrichtung (100) nach einem der Ansprüche 1 bis 13 in das Reservoir (103) der Vorrichtung,
b) Mischen des Wirkstoffs (104) innerhalb des Reservoirs (103) mit der in Schritt a) aufgenommenen Flüssigkeit (202),
c) dadurch Erhalt einer antiseptischen Wundspüllösung,
d) gegebenenfalls Abgabe der antiseptischen Wundspüllösung in den Behälter (200).

15. Verwendung einer Vorrichtung (100) nach einem der Ansprüche 1 bis 12 zur Herstellung einer antiseptischen Wundspüllösung.

## Claims

1. A device (100) for producing an antiseptic wound irrigation solution, comprising
- a connection element (101) which is designed and configured for liquid-tight connection to a container (200);
- a mandrel (102) which is designed and configured for piercing a septum (201) of a container;
- a reservoir (103) which contains an active ingredient (104) in solid form, and can be connected via an outlet (105) of the reservoir in a fluid-conducting manner to the connection element (101);
- a liquid-permeable filter (106) which is arranged at the outlet (105) of the reservoir, and is configured to retain the active ingredient (104) in solid form in the reservoir (103);
- a fluidic element (107) which is configured to receive a liquid (202) from a container (200) into the reservoir (103), and/or is configured to dispense a liquid (202) from the reservoir (103);
wherein the device (100) is designed and configured to receive a liquid (202) from a container (200) into the reservoir, to dissolve the active ingredient (104) in the liquid (202), and to dispense the dissolved active ingredient to a patient via the connection element (101),
wherein the active ingredient is an antiseptic, which is selected from the group consisting of calcium hypochlorite, magnesium hypochlorite, sodium hypochlorite, sodium percarbonate, sodium peroxide, calcium peroxide, urea peroxide, triisocyanuric chloride, sodium-N-chloro-4-methylbenzene sulfonamide, sodium-N-chlorobenzene sulfonamide, octenidine, polyhexanide, and chlorhexidine digluconate.

2. The device according to claim 1, wherein the mandrel (102) has a hollow interior (115) which is configured to receive a liquid (202) from a container (200) into the reservoir (103).

3. The device according to either of the preceding claims, wherein the fluidic element (107) comprises a plunger (108) which is movably arranged within the reservoir (103) and closes off the reservoir in a liquid-tight manner.

4. The device according to any of the preceding claims, which comprises a first component (150) and a second component (160) which can be detachably connected to one another.

5. The device according to claim 4, wherein the first component (150) contains the connection element (101) and the mandrel (102), and/or wherein the second component (160) contains the reservoir (103), the filter (106), and the fluidic element (107).

6. The device according to claim 4 or claim 5, wherein the device also comprises a connecting element (109) by means of which the first component (150) can be connected to the second component (160).

7. The device according to any of the preceding claims, wherein the device is completely sterilized, and preferably is completely non-pyrogenic.

8. The device according to any of the preceding claims, wherein the device also comprises a nozzle (110) which is configured to dispense the active ingredient in dissolved form.

9. The device according to claim 8, wherein the nozzle (110) can be detachably connected in a fluid-conducting manner to the connection element (101).

10. The device according to any of the preceding claims, wherein the connection element (101) comprises a first feedthrough (111) which is configured to receive a liquid (202) from a container (200) into the reservoir (103).

11. The device according to any of the preceding claims, wherein the connection element (101) comprises a second feedthrough (112) which is configured to dispense a liquid (202) from a container (200), wherein the dispensing is preferably possible in a state in which the device (100) is connected to the reservoir (103) via the first feedthrough (111) according to claim 10.

12. The device according to any of the preceding claims, wherein the reservoir (103) is permeable to gamma rays and/or electron beams.

13. A kit, comprising a device (100) according to any of claims 1 to 12 and a container (200) having a medically compatible liquid, wherein the device (100) can be detachably connected to the container (200), wherein the kit is packed together in a germ-tight manner, preferably is completely sterilized, and more preferably is completely non-pyrogenic.

14. A method for producing an antiseptic would irrigation solution which comprises the following steps
a) receiving a liquid (202) from a container (200) using a device (100) according to any of claims 1 to 13 into the reservoir (103) of the device,
b) mixing the active ingredient (104) within the reservoir (103) with the liquid (202) received in step a),
c) thereby obtaining an antiseptic wound irrigation solution,
d) optionally dispensing the antiseptic wound irrigation solution into the container (200).

15. The use of a device (100) according to any of claims 1 to 12 for producing an antiseptic wound irrigation solution.

## Revendications

1. Dispositif (100) pour la préparation d'une solution de rinçage de plaies antiseptique, présentant
- un élément de raccordement (101) qui est réalisé et conçu pour le raccordement imperméable aux liquides à un récipient (200) ;
- un mandrin (102) qui est réalisé et conçu pour perforer une cloison (201) d'un récipient ;
- un réservoir (103) qui contient une substance active (104) sous forme solide et qui peut être relié d'une manière conductrice de fluide à l'élément de raccordement (101) par l'intermédiaire d'une sortie (105) du réservoir ;
- un filtre (106) perméable aux liquides qui est disposé au niveau de la sortie (105) du réservoir et qui est conçu pour retenir la substance active (104) sous forme solide dans le réservoir (103) ;
- un élément fluidique (107) qui est conçu pour recevoir un liquide (202) provenant d'un récipient (200) dans le réservoir (103) et/ou pour distribuer un liquide (202) provenant du réservoir (103) ;
dans lequel le dispositif (100) est réalisé et conçu pour recevoir un liquide (202) provenant d'un récipient (200) dans le réservoir, pour dissoudre la substance active (104) dans le liquide (202), et pour distribuer la substance active dissoute à un patient par l'intermédiaire de l'élément de raccordement (101),
dans lequel la substance active est un antiseptique qui est choisi dans le groupe constitué d'hypochlorite de calcium, hypochlorite de magnésium, hypochlorite de sodium, percarbonate de sodium, peroxyde de sodium, peroxyde de calcium, peroxyde d'urée, chlorure de triisocyanure, N-chloro-4-méthylbenzènesulfonamide de sodium, N-chlorobenzènesulfonamide de sodium, octénidine, polyhexanide et digluconate de chlorhexidine.

2. Dispositif selon la revendication 1, dans lequel le mandrin (102) présente un espace intérieur creux (115) qui est conçu pour recevoir un liquide (202) provenant d'un récipient (200) dans le réservoir (103).

3. Dispositif selon l'une des revendications précédentes, dans lequel l'élément fluidique (107) présente un piston (108) disposé de manière mobile à l'intérieur du réservoir (103) et fermant le réservoir de manière imperméable aux liquides.

4. Dispositif selon l'une des revendications précédentes, lequel présente un premier composant (150) et un second composant (160) qui peuvent être reliés l'un à l'autre de manière amovible.

5. Dispositif selon la revendication 4, dans lequel le premier composant (150) contient l'élément de raccordement (101) et le mandrin (102), et/ou dans lequel le second composant (160) contient le réservoir (103), le filtre (106) et l'élément fluidique (107).

6. Dispositif selon la revendication 4 ou 5, dans lequel le dispositif présente en outre un élément de liaison (109) à l'aide duquel le premier composant (150) peut être relié au second composant (160).

7. Dispositif selon l'une des revendications précédentes, dans lequel le dispositif est entièrement stérilisé, de préférence est entièrement apyrogène.

8. Dispositif selon l'une des revendications précédentes, dans lequel le dispositif présente en outre une buse (110) qui est conçue pour distribuer la substance active sous forme dissoute.

9. Dispositif selon la revendication 8, dans lequel la buse (110) peut être reliée de manière amovible et conductrice de fluide à l'élément de raccordement (101).

10. Dispositif selon l'une des revendications précédentes, dans lequel l'élément de raccordement (101) présente un premier passage (111) qui est conçu pour recevoir un liquide (202) provenant d'un récipient (200) dans le réservoir (103).

11. Dispositif selon l'une des revendications précédentes, dans lequel l'élément de raccordement (101) présente un second passage (112) qui est conçu pour distribuer un liquide (202) à partir d'un récipient (200), dans lequel la distribution est de préférence possible dans un état dans lequel le dispositif (100) est relié au réservoir (103) par l'intermédiaire du premier passage (111) conformément à la revendication 10.

12. Dispositif selon l'une des revendications précédentes, dans lequel le réservoir (103) est perméable aux rayons gamma et/ou aux faisceaux électroniques.

13. Kit, présentant un dispositif (100) selon l'une des revendications 1 à 12 et un récipient (200) comportant un liquide médicalement compatible, dans lequel le dispositif (100) peut être relié de manière amovible au récipient (200), dans lequel le kit est emballé conjointement de manière imperméable aux germes, de préférence est entièrement stérilisé, plus préférentiellement est entièrement apyrogène.

14. Procédé pour la préparation d'une solution de rinçage de plaies antiseptique, lequel comprend les étapes suivantes,
a) réception d'un liquide (202) provenant d'un récipient (200) à l'aide d'un dispositif (100) selon l'une des revendications 1 à 13 dans le réservoir (103) du dispositif,
b) mélange de la substance active (104) à l'intérieur du réservoir (103) avec le liquide (202) reçu à l'étape a),
c) obtention consécutive d'une solution de rinçage de plaies antiseptique,
d) éventuellement, distribution de la solution de rinçage de plaies antiseptique dans le récipient (200).

15. Utilisation d'un dispositif (100) selon l'une des revendications 1 à 12 pour la préparation d'une solution de rinçage de plaies antiseptique.
